(19) Europäisches Patentamt
European Patent Office
Office européen des brevets

(11) EP 1 607 083 A1

(12) **DEMANDE DE BREVET EUROPEEN**

(43) Date de publication:
**21.12.2005 Bulletin 2005/51**

(51) Int Cl.[7]: **A61K 7/02**, A61K 7/40, A61K 7/06

(21) Numéro de dépôt: **05291275.5**

(22) Date de dépôt: **14.06.2005**

(84) Etats contractants désignés:
**AT BE BG CH CY CZ DE DK EE ES FI FR GB GR HU IE IS IT LI LT LU MC NL PL PT RO SE SI SK TR**
Etats d'extension désignés:
**AL BA HR LV MK YU**

(30) Priorité: **16.06.2004 FR 0406539**

(71) Demandeur: **L'OREAL**
**75008 Paris (FR)**

(72) Inventeurs:
- **Seyler, Nathalie**
**94700 Maisons-Alfort (FR)**
- **Guiramand, Carole**
**78350 Jouy en Josas (FR)**
- **Ribaud, Christèle**
**94130 Nogent S/Marne (FR)**

(74) Mandataire: **Allab, Myriam**
**L'OREAL - D.I.P.I.**
**25-29 Quai Aulagnier**
**92600 Asnières (FR)**

(54) **Procédé pour favoriser la pénétration d'un actif cosmétique et composition permettant sa mise en oeuvre**

(57) L'invention concerne un procédé pour favoriser la pénétration d'un actif cosmétique caractérisé en ce que l'on applique topiquement une composition sous forme d'une émulsion eau dans huile, ladite émulsion comprenant une phase grasse de polarité δa supérieure ou égale à 0,1 et comportant un mélange d'au moins deux huiles, et en ce que l'application de ladite émulsion est réalisée de façon simultanée, décalée ou séquencée dans le temps avec l'application de l'actif cosmétique.

EP 1 607 083 A1

## Description

La présente invention concerne un procédé pour favoriser la pénétration d'un actif cosmétique dans la peau et/ou le cuir chevelu, et donc améliorer son activité en augmentant la vitesse et/ou la quantité d'actif traversant la couche cornée pour atteindre son site d'action. Elle concerne également des compositions pouvant être utilisées pour améliorer la pénétration d'un actif cosmétique ou dermatologique.

La peau est constituée de deux compartiments, l'un superficiel, l'épiderme, et un plus profond, le derme, qui interagissent. L'épiderme humain naturel est composé principalement de trois types de cellules qui sont les kératinocytes, très majoritaires, les mélanocytes et les cellules de Langerhans. Chacun de ces types cellulaires contribue par ses fonctions propres au rôle essentiel joué dans l'organisme par la peau, notamment le rôle de protection de l'organisme des agressions extérieures appelé "fonction barrière".

L'épiderme est conventionnellement divisé en une couche basale de kératinocytes constituant la couche germinative de l'épiderme, une couche dite épineuse constituée de plusieurs couches de cellules polyédriques disposées sur les couches germinatives, une à trois couches dites granuleuses constituées de cellules aplaties contenant des inclusions cytoplasmiques distinctes, les grains de kératohyaline et enfin la couche cornée (ou *stratum corneum*), constituée d'un ensemble de couches de kératinocytes au stade terminal de leur différenciation appelés cornéocytes.

Le derme fournit à l'épiderme un support solide. C'est également son élément nourricier. Il est principalement constitué de fibroblastes et d'une matrice extracellulaire composée majoritairement de collagène, d'élastine et d'une substance, dite substance fondamentale. Ces composants sont synthétisés par les fibroblastes. On y trouve aussi des leucocytes, des mastocytes ou encore des macrophages tissulaires. Enfin, le derme est traversé par des vaisseaux sanguins et des fibres nerveuses.

La cohésion entre l'épiderme et le derme est assurée par la jonction dermo-épidermique.

L'administration de composés actifs par voie topique se heurte très souvent à un problème de pénétration de la couche cornée ou stratum corneum de telle sorte que ceux-ci ne peuvent atteindre des couches plus profondes de la peau, l'épiderme et le derme.

Différentes solutions ont été proposées pour améliorer la perméabilité cutanée pour des formulations contenant des agents actifs sur des couches sous-jacentes de l'épiderme ou du derme. Cependant il existe toujours un besoin de formulations cosmétiques bien tolérées et efficaces pour lutter contre cet effet barrière du stratum corneum à la diffusion des actifs.

US 2003/0017176 décrit des compositions de type émulsion eau dans huile, présentant une faible viscosité et contenant une forte teneur en eau, comprenant obligatoirement des tensioactifs siliconés.

EP 1342463 décrit des compositions pour les lèvres telles que des sticks, permettant de masquer le goût amer du filtre solaire bis-ethyloxyphénol méthoxyphényl triazine; on sait qu'il est souhaitable que les filtres UV demeurent à la surface cutanée.

US 2003/0108579 décrit des compositions anhydres comprenant au moins un éther de polyol, une huile et une cire, qui sont solides à 37°C.

US 5,489,429 décrit des émulsions eau dans huile pour le soin des mains comprenant un ester mixte d'acide isostéarique et d'acide succinique avec la glycérine, et au moins 10% en poids de polyol.

De manière inattendue, la demanderesse a trouvé que le traitement ou le prétraitement de la surface cutanée par une composition cosmétique présentant une phase huileuse polaire comportant au moins deux huiles favorise la pénétration cutanée d'actifs.

C'est pourquoi la présente invention a pour objet un procédé pour favoriser la pénétration d'un actif cosmétique, caractérisé en ce que l'on applique topiquement une composition sous forme d'une émulsion eau dans huile, ladite émulsion comprenant une phase grasse de polarité δa supérieure ou égale à 0,1 et comportant un mélange d'au moins deux huiles, et en ce que l'application de ladite émulsion est réalisée de façon simultanée, décalée ou séquencée dans le temps avec l'application de l'actif cosmétique.

La polarité peut être décrite par le paramètre de solubilité de Hansen δa, elle est exprimée en $J^{1/2}\ cm^{-3/2}$ dans tout ce texte, à moins qu'il ne soit spécifié différemment. En effet, ce paramètre caractérise, pour un constituant donné, l'énergie correspondant aux interactions polaires (δp) et de types liaisons hydrogène (δh) existant entre les molécules de ce constituant.

$$\delta_a = \sqrt{\delta_p^2 + \delta_h^2}$$

Tel qu'il est rappelé dans l'ouvrage « Properties of Polymers » de D.W.Van Krevelen 3th edition (Elsevier, 1990) page 200 et sq, la solubilité d'un composé dans un solvant donné est en grande partie déterminée par sa structure chimique.

Les huiles apolaires ont une valeur de δa égale à 0.

Les huiles polaires ont une valeur de δa différente de 0, c'est-à-dire supérieure à 0.
La composition sous forme d'émulsion eau dans huile (E/H) est appliquée sur la peau et/ou le cuir chevelu avant ou en même temps que le principe actif dont la pénétration doit être améliorée, de façon à augmenter la quantité d'actif atteignant son site d'action et/ou la vitesse de pénétration de l'actif, et donc sa biodisponibilité.
Le terme actif cosmétique désigne dans le présent texte un composé ou un mélange de composés, sous forme purifiée ou sous forme de complexe, notamment minéral ou végétal, présentant une activité intrinsèque *in vitro* ou *in vivo*, et pouvant être formulé dans un produit cosmétique.
Par "produit cosmétique", on entend notamment toute substance ou préparation destinée à être mise en contact avec les diverses parties superficielles du corps humain (épiderme, système pileux et capillaire, ongles, lèvres et organes génitaux externes) ou avec les dents et les muqueuses buccales en vue, exclusivement ou principalement, de les nettoyer, de les parfumer, d'en modifier l'aspect et/ou de corriger les odeurs corporelles et/ou de les protéger ou de les maintenir en bon état (directive cosmétique 76/768/CEE modifiée).
La procédé selon l'invention sera bien évidemment également adapté pour favoriser la pénétration de plusieurs actifs.

Selon un mode de réalisation de l'invention, l'émulsion E/H comprend un mélange d'au moins trois huiles, la phase grasse présentant une valeur finale de δa supérieure ou égale à 0,1.
De préférence, la polarité finale de la phase grasse est caractérisée par une valeur de δa supérieure ou égale à 0,2, notamment supérieure ou égale à 0,4.
Généralement, la polarité finale de la phase grasse de l'émulsion correspond à une valeur de δa inférieure ou égale à 8, notamment inférieure ou égale à 5, voire inférieure ou égale à 4.

La phase grasse de l'émulsion peut comprendre un mélange d'huiles apolaires et d'huiles polaires, dont les quantités respectives seront adaptées par l'homme du métier pour obtenir une phase grasse dont les valeurs de δa sont conformes à l'invention.
Par exemple, la phase grasse de l'émulsion comprend de 20% à 80%, par rapport au poids total de la phase grasse, en particulier de 40% à 75% d'huiles polaires possédant des δa compris entre 2 et 11. Selon un mode de réalisation de l'invention, les huiles polaires présentes dans la phase grasses ont des valeurs de δa propres de 3 à 8, notamment supérieures à 3,9.
Selon l'un des modes de réalisation avantageux de l'invention, les huiles de la phase grasse, comprennent au moins une huile de viscosité inférieure ou égale à 10cst.

De préférence, les compositions selon l'invention sont liquides ou fluides à 37°C, voire à température ambiante, soit environ 25°C.

Parmi les huiles présentes dans la composition, on peut citer les huiles de type silicone et/ou de type huile de synthèse ou ester de synthèse et/ou de type huile végétale à forte teneur en triglycérides; on peut choisir les huiles hydrocarbonées, les huiles siliconées et/ou les huiles fluorées.
En particulier, l'émulsion comprendra au moins une huile polaire choisie parmi les huiles végétales à forte teneur en triglycérides, les huiles de synthèse et/ou esters de synthèse et les huiles siliconées.
Par "huile", on entend tout milieu non aqueux liquide à température ambiante (25°C) et pression atmosphérique (760 mm de Hg), physiologiquement acceptable.

Ces huiles peuvent être des huiles hydrocarbonées et/ou siliconées et/ou fluorées. Elles peuvent être d'origine animale, végétale, minérale ou synthétique. On entend par « huile hydrocarbonée », toute huile comportant majoritairement des atomes de carbone et d'hydrogène, et éventuellement des groupements ester, éther, fluoré, acide carboxylique et/ou alcool. En outre, les huiles utilisées peuvent être volatiles et/ou non volatiles. Par huile volatile, on entend une huile susceptible de s'évaporer à température ambiante d'un support sur lequel elle a été appliquée, autrement dit une huile ayant une tension de vapeur mesurable à 25°C et 1 atmosphère, par exemple supérieure à 0 Pa, en particulier allant de 10-3 à 300 mm de Hg (0,13 Pa à 40.000 Pa). On peut notamment citer comme huiles volatiles, les huiles siliconées volatiles, telles que les silicones volatiles cycliques ou linéaires. On peut également citer les huiles volatiles hydrocarbonées telles que les isoparaffines, et les huiles fluorées volatiles.

Parmi les huiles pouvant être utilisées dans la composition de l'invention, certaines sont polaires et d'autres sont apolaires (c'est-à-dire non polaires).

Les huiles polaires comportent dans leur structure chimique au moins un groupement polaire non ionique, et de préférence au moins deux groupements polaires non ioniques ou ioniques tels que les groupements suivants :

- COOH;
- OH mono ou disubstitué (primaire ou secondaire) ;
- PO4;
- NHR ; NR1R2, R1 et R2 formant éventuellement un cycle et représentant un radical alkyle ou alcoxy linéaire ou ramifié en C1 à C20, ou

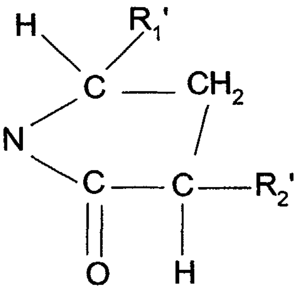

où R1' et R2' peuvent représenter l'hydrogène ou une chaîne alkyle ou alkoxy linéaire ou ramifiée en C1 à C20. Les huiles apolaires ont une valeur de δa égale à 0. En particulier, les huiles apolaires selon l'invention peuvent être en particulier choisies parmi :

- les hydrocarbures, linéaires ou ramifiés, d'origine synthétique ou minérale, comme les huiles de paraffine, volatiles ou non volatiles, et leurs dérivés ; l'huile de vaseline ; la lanoline liquide ; les polydécènes ; le polyisobutène hydrogéné tel que l'huile de Parléam® ; le squalane ; l'isoparaffine hydrogénée ; l'isohexadecane ; l'isododecane ;
- et leurs mélanges.

Les huiles polaires ont une valeur de δa différente de 0, c'est-à-dire supérieure à 0. En particulier, les huiles polaires utilisées dans la composition de l'invention peuvent être choisies parmi :

- les huiles d'origine végétale, huiles hydrocarbonées à forte teneur en triglycérides constitués d'esters d'acides gras et de glycérol dont les acides gras peuvent avoir des longueurs de chaînes variées de C4 à C24, ces dernières pouvant être linéaires ou ramifiées, saturées ou insaturées. Comme huiles d'origine végétale, on peut citer notamment les huiles de jojoba, de germes de blé, de maïs, de tournesol, de beurre de karité, de ricin, d'amandes douces, de macadamia, d'abricot, de soja, de coton, de luzerne, de pavot, de potimarron, de sésame, de courge, de colza, d'avocat, de noisette, de pépins de raisin ou de cassis, d'onagre, de millet, d'orge, de quinoa, d'olive, de seigle, de carthame, de bancoulier, de passiflore, de rosier muscat, de coriandre ; ou encore les triglycérides des acides caprylique/caprique comme ceux vendus par la société Stearineries Dubois ou ceux vendus sous les dénominations Miglyol 810, 812 et 818 par la société Dynamit Nobel ;
- les huiles de synthèse ou esters de synthèse de formule R5COOR6 dans laquelle R5 représente le reste d'un acide gras linéaire ou ramifié comportant de 1 à 40 atomes de carbone et R6 représente une chaîne hydrocarbonée notamment ramifiée contenant de 1 à 40 atomes de carbone à condition que R5 + R6 soit ≥ 10, comme par exemple l'huile de Purcellin (octanoate de cétostéaryle), l'isononanoate d'isononyle, le benzoate d'alcool en C12 à C15, le myristate d'isopropyle, le palmitate d'éthyl 2-hexyle, l'isostéarate d'isostéaryle, l'isostéarate d'isopropyle, des octanoates, décanoates ou ricinoléates d'alcools ou de polyalcools ; les esters hydroxylés comme le lactate d'isostéaryle, le malate de di-isostéaryle ; ,le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, les esters de polyol, comme le dioctanoate de propylène glycol, le diheptanoate de néopentylglycol et le diisononanoate de diéthylèneglycol ; les esters du pentaérythritol comme le tétraisostéarate de pentaérythrytyle ; les dérivés lipophiles d'acides aminés, tels que le lauroyl sarcosinate d'isopropyle (nom INCI : Isopropyl Lauroyl sarcosinate) commercialisé sous la dénomination Eldew SL 205 par la société Ajinomoto ;;
- les éthers de synthèse ayant de 10 à 40 atomes de carbone ;
- les alcools gras en C8 à C26, comme l'alcool oléique, l'alcool isostéarylique, et l'octyldodécanol ;
- les huiles de silicone, telles que les polydiméthylsiloxanes (PDMS) volatils ou non, linéaires ou cycliques, liquides à température ambiante ; les silicones phénylées comme les phényltriméthicones, les phényldiméthicones, les phényl-triméthyl-siloxydiphénylsiloxanes, des diphényldiméthicones, les diphényl-méthyl-diphényl-trisiloxanes, les 2-phényléthyl triméthylsiloxysilicates, les polyméthyl-phénylsiloxanes et les silicones alkylées ;
- leurs mélanges.

Toutefois, selon l'un des aspects de l'invention, les alcools gras utilisables ne comprennent pas les alcools gras en C18 insaturés.

Parmi les silicones, on choisira préférentiellement les diméthicones. Parmi les huiles ou esters de synthèse

on préfèrera les huiles ou esters de synthèse de formule $R_5COOR_6$ dans laquelle $R_5$ représente le reste d'un acide gras linéaire ou ramifié comportant de 1 à 40 atomes de carbone et $R_6$ représente une chaîne hydrocarbonée notamment ramifiée contenant de 1 à 40 atomes de carbone à condition que $R_5$ + $R_6$ soit > ou = à 10. On préfèrera en particulier l'isononanoate d'isononyle et le lauroyl sarcosinate d'isopropyle. Parmi les huiles végétales à forte teneur en triglycérides, on choisira préférentiellement l'huile d'amandes d'abricot.

La composition sera préparée de façon connue par l'homme du métier, la quantité de la phase grasse dans l'émulsion pouvant varier, notamment de 2,5% à 50% en poids par rapport au poids total de la composition, notamment de 4 à 40%.

Les compositions de l'invention peuvent comprendre en outre des adjuvants cosmétiques classiques notamment choisis parmi les corps gras, les solvants organiques, les épaississants, les adoucissants, les opacifiants, les stabilisants, les émollients, les agents anti-mousse, les agents hydratants, les parfums, les conservateurs, les polymères, les charges, les séquestrants, les propulseurs, les agents alcalinisants ou acidifiants ou tout autre ingrédient habituellement utilisé en cosmétique.

En particulier, la composition E/H contient un émulsionnant (ou tensioactif), en une quantité par exemple de 0,1 à 40% en poids par rapport au poids total de la composition, notamment de 0,5 à 20 %.

Des émulsions eau dans huile peuvent être stabilisées par différents tensioactifs, en particulier, des dérivés alkylés de polyglycérol, des polyéthylène glycols alkylés, des dérivés alkylés de sorbitane, des sels métalliques d'acides gras, des tensioactifs siliconés et préférentiellement par des oligomères ou polymères dérivés de polyoléfines.

Dans un des modes de réalisation, le tensioactif est choisi dans le groupe comprenant les dérivés alkylés de polyglycérol, les polyéthylènes glycol alkylés, les dérivés alkylés de sorbitane, les sels métalliques d'acides gras, et les oligomères ou polymères dérivés de polyoléfines. En particulier, les tensioactifs siliconés peuvent être essentiellement absents de la composition destinée à favoriser la pénétration selon l'invention.

On peut citer par exemple comme émulsionnants les dimethicone copolyols tels que le mélange de cyclomethicone et de dimethicone copolyol, vendu sous la dénomination « DC 5225 C » par la société Dow Corning, et les alkyldimethicone copolyols tels que le Laurylmethicone copolyol vendu sous la dénomination "Dow Corning 5200 Formulation Aid" par la société Dow Corning et le Cetyl dimethicone copolyol vendu sous la dénomination ABIL EM 90R par la société Goldschmidt, ou le mélange polyglycéryl-4 isostéarate/cétyl diméthicone copolyol/hexyllaurate vendu sous la dénomination ABIL WE 09 par la société Goldschmidt.

On peut y ajouter aussi un ou plusieurs co-émulsionnants. De manière avantageuse, le co-émulsionnant peut être choisi dans le groupe comprenant les esters alkylés de polyol. Comme esters alkylés de polyol, on peut citer notamment les esters de glycérol et/ou de sorbitan et par exemple l'isostéarate de polyglycérol, tel que le produit commercialisé sous la dénomination Isolan GI 34 par la société Goldschmidt, l'isostéarate de sorbitan, tel que le produit commercialisé sous la dénomination Arlacel 987 par la société ICI, l'isostéarate de sorbitan et de glycérol, tel que le produit commercialisé sous la dénomination Arlacel 986 par la société ICI, et leurs mélanges.

On peut aussi utiliser comme tensioactif d'émulsions E/H, un organopolysiloxane solide élastomère réticulé comportant au moins un groupement oxyalkyléné, tel que ceux obtenus selon le mode opératoire des exemples 3, 4 et 8 du document US-A-5,412,004 et des exemples du document US-A-5,811,487, notamment le produit de l'exemple 3 (exemple de synthèse) du brevet US-A-5,412,004. et tel que celui commercialisé sous la référence KSG 21 par la société Shin Etsu.

Des oligomères et polymères dérivés de polyoléfines utiles comme émulsionnants dans la composition de l'invention sont constitués d'une partie apolaire polyoléfinique et d'au moins une partie polaire. Ils peuvent présenter une structure de type bloc ou peigne. La partie apolaire polyoléfinique comprend au moins 40 atomes de carbone et de préférence de 60 à 700 atomes de carbone. Cette partie apolaire peut être choisie parmi les polyoléfines telles que les oligomères, les polymères et/ou les copolymères d'éthylène, d'éthylène, de propylène, de 1-butène, d'isobutène, de 1-pentène, de 2-méthyl-1-butène, de 3-methyl-1-butène, de 1-héxène, de 1-heptène, de 1-octène, de 1-décène, de 1-undécène, de 1-dodécéne, de 1-tridécène, de 1-tetradécène, de 1-pentadécène, de 1-hexadécéne, de 1-heptadécène et de 1-octadécéne. Ces polyoléfines sont hydrogénées ou non.

Par ailleurs, les oligomères ou polymères dérivés de polyoléfine utilisés dans la composition de l'invention comportent au moins une partie polaire. Cette partie polaire confère aux dérivés de polyoléfines des propriétés amphiphiles. Ainsi, ces oligomères ou polymères abaissent la tension interfaciale (eau / huile, c'est-à-dire entre phase aqueuse et phase huileuse) d'au moins 10 mN/m quand ils sont présents à une concentration de 0,01% en poids par rapport au poids total de la phase huileuse.

La partie polaire des émulsionnants oligomères ou polymères de l'invention peut être anionique, cationique, non ionique, zwitterionique ou amphotère. Elle est par exemple constituée de polyalkylène glycols ou de polyalkylène imines, ou encore d'acides ou de diacides carboxyliques, de leurs anhydrides ou de leurs dérivés, et leurs mélanges.

Des émulsionnants oligomères ou polymères à partie polaire acide carboxylique peuvent être par exemple issus de la réaction entre une polyoléfine et au moins un acide ou anhydride carboxylique choisi dans le groupe comprenant l'acide maléique, l'anhydride maléique, l'acide fumarique, l'acide itaconique, l'acide citraconique, l'acide mésaconique,

l'acide aconitique. De préférence, la partie polaire est constituée par l'acide ou l'anhydride succinique, leurs dérivés esters ou amides, les sels d'ions alcalins, alcalino-terreux ou organiques correspondants, ou bien encore par du polyoxyéthylène.

Les émulsionnants dérivés de polyoxyéthylène peuvent être par exemple choisis parmi les polymères diblocs polyisoprène-polyoxyéthylène, les polymères poly(éthylène-co-propylène)-polyoxyéthylène et leurs mélanges. Ces polymères sont décrits dans la publication de Allgaier, Poppe, Willner, Richter (Macromolecules, 1997, vol. 30, p. 1582-1586).

Les émulsionnants dérivés d'acide ou d'anhydride succinique peuvent être choisis notamment parmi les dérivés polyoléfines d'acide ou d'anhydride succinique décrits dans les brevets US-A-4,234,435, US-A-4,708,753, US-A-5,129,972, US-A-4,931,110, GB-A-2,156,799 et US-A-4,919,179 incorporés ici pour référence. La partie polyoléfine peut être constituée par exemple de polyisobutylène, hydrogéné ou non, de poids moléculaire allant de 400 à 5000. Dans le polyisobutylène à terminaison succinique ainsi obtenu, la partie succinique peut être estérifiée, amidifiée ou sous forme de sel, c'est-à-dire qu'elle peut être modifiée par des alcools, des amines, des alcanolamines ou des polyols, ou encore se trouver sous forme de sels de métal alcalin ou alcalinoterreux, d'ammonium ou encore de base organique comme les sels de diéthanolamine et de triéthanolamine. Les polyoléfines à terminaison succinique estérifiée ou amidifiée sont des produits de réaction de (a) une polyoléfine à terminaison succinique, et de (b) une amine ou un alcool, pour former une amide ou un ester. Le terme « amine » utilisé ici comprend tous types d'amines dont les alcanolamines. Il peut s'agir par exemple de mono-amines primaires, secondaires ou tertiaires, ces amines pouvant être aliphatiques, cycloaliphatiques, aromatiques, hétérocycliques, saturées ou insaturées. Par ailleurs, les alcools peuvent être des mono- ou poly-alcools. Les mono-alcools comprennent les alcools aliphatiques primaires, secondaires ou tertiaires, et les phénols. Les polyalcools peuvent être par exemple choisis parmi les poly-alcools aliphatique, cycloaliphatiques, aromatiques et hétérocycliques. Les polyoléfines à terminaison succinique modifiée (estérifiée ou amidifiée) et leur procédé de préparation sont décrits en particulier dans le document US-A-4,708,753 qui est incorporé ici pour référence.

Des oligomères ou polymères dérivés de polyoléfines adaptés à l'invention sont avantageusement des polyoléfines à terminaison succinique; on peut citer notamment les polyisobutylène à terminaison succinique modifiée, tels que les produits commercialisés sous les dénominations L2724, L2721, Lubrizol 5603® par la société Lubrizol, l'Hostacerin® PIB par la société Clariant et le Chemcinnate 2000® par la société Chemron.

Un autre exemple de tensioactif polymérique utilisable dans l'invention est le produit de la réaction de l'anhydride maléique avec le polyisobutylène, tel que le produit commercialisé sous la dénomination Glissopal SA par la société BASF. La quantité d'oligomère(s) ou de polymère(s) émulsionnant(s) dans la composition de l'invention peut aller par exemple de 0,1 à 10 % en poids de matière active, de préférence de 0,5 à 5 % en poids et mieux de 1 à 3 % en poids par rapport au poids total de la composition. On peut utiliser un ou plusieurs oligomères ou polymères dérivés de polyoléfines. Selon un mode préféré de réalisation de l'invention, les oligomères ou polymères dérivés de polyoléfines sont les seuls émulsionnants utilisés dans la composition selon l'invention. Toutefois, on peut éventuellement, ajouter d'autres agents amphiphiles habituellement utilisés dans les émulsions eau-dans-huile, tels que les tensioactifs classiques ioniques, non ioniques, amphotères, zwitterioniques, les oligomères ou les polymères amphiphiles, les particules organiques ou inorganiques amphiphiles.

Selon l'un des modes de réalisation de l'invention, les compositions destinées à favoriser la pénétration sont essentiellement exemptes d'acide isostéarique, et/ou les tensioactifs ne sont pas co-émulsionnés avec l'acide succinique.

L'un des objets de l'invention est un procédé pour favoriser la pénétration d'un actif cosmétique tel que défini précédemment, qui comprend les étapes suivantes:

(a) on applique l'émulsion eau-dans-huile sur une zone de la peau et/ou du cuir chevelu,
(b) on laisse en contact l'émulsion avec la peau et/ou le cuir chevelu pendant un temps supérieur ou égal à 2 minutes,
(c) on applique l'actif cosmétique sur la même zone de la peau et/ou du cuir chevelu que celle de l'étape (a).

Selon l'un des modes de réalisation de l'invention, les étapes (a) et (c) sont effectuées de façon séquencée dans le temps, et l'actif cosmétique est présent dans une composition cosmétique comprenant un milieu physiologiquement acceptable.

Selon un autre mode de réalisation de l'invention, les étapes (a) et (c) sont effectuées simultanément, et l'actif cosmétique est présent dans la composition sous forme d'émulsion eau dans huile de l'étape (a).

Par "physiologiquement acceptable", on entend un milieu compatible avec la peau, les muqueuses, les lèvres et/ou les phanères, notamment les ongles ou les cheveux; de préférence, le milieu sera cosmétiquement acceptable, c'est-à-dire qu'il présente un aspect, un toucher, une odeur et éventuellement un goût agréable pour l'utilisateur.

Les compositions de l'étape (a) comprennent bien évidemment un support cosmétiquement ou dermatologiquement acceptable. Dans le cas où l'étape (c) est réalisée de façon décalée par rapport à l'étape (a), la composition

de l'étape (c) peut se présenter sous toutes les formes galéniques normalement utilisées pour une application topique, notamment sous forme d'une solution aqueuse, hydroalcoolique ou huileuse, d'une émulsion huile-dans-eau ou eau-dans-huile ou multiple, d'un gel aqueux ou huileux, d'un produit anhydre liquide, pâteux ou solide, d'une dispersion d'huile dans une phase aqueuse à l'aide de sphérules, ces sphérules pouvant être des nanoparticules polymériques telles que les nanosphères et les nanocapsules ou mieux des vésicules lipidiques de type ionique et/ou non-ionique.

La composition utilisable aux étapes (a) et /ou (c) peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sur la peau sous forme d'aérosol. Elle peut également se présenter sous forme solide, et par exemple sous forme de stick. Elle peut être utilisée comme produit de soin, comme produit de nettoyage, comme produit de maquillage ou encore comme simple produit déodorant.

De façon connue, la composition de l'invention peut contenir également les adjuvants habituels dans les domaines cosmétiques et dermatologique, tels que les gélifiants hydrophiles ou lipophiles, les actifs hydrophiles ou lipophiles, les conservateurs, les antioxydants, les solvants, les parfums, les charges, les filtres, les pigments, les agents chélateurs, les absorbeurs d'odeur et les matières colorantes. Les quantités de ces différents adjuvants sont celles classiquement utilisées dans les domaines considérés, et par exemple de 0,01% à 20 % du poids total de la composition. Ces adjuvants, selon leur nature, peuvent être introduits dans la phase grasse, dans la phase aqueuse, dans les vésicules lipidiques et/ou dans les nanoparticules.

Les compositions selon l'invention peuvent contenir des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP-A-0518772 et EP-A-0518773.

Toutefois, lorsque des filtres UV sont présents dans la composition, de préférence leur poids moléculaire est supérieur ou égal à 500, et/ou ils sont associés à des structures telles que des microcapsules pour limiter leur passage transcutané. Selon un mode de réalisation particulier, la composition ne contient pas de bis-ethyloxyphénol méthoxyphényl triazine, ou si ce filtre est présent, il est associé au moins à un filtre UV différent.

Le temps de contact de l'étape (b) sera adapté pour obtenir le degré et la vitesse de pénétration convenant aux principes actifs et à l'activité cosmétique visés dans un mode de réalisation choisi.

Par exemple, l'émulsion E/H sera appliquée sur l'ensemble du visage et du cou sous forme d'un masque ou d'une lotion de prétraitement pendant une durée de 2 à 20 minutes, notamment d'environ 5 à 10 minutes, mais cette durée pourra être prolongée sans inconvénient, par exemple jusqu'à 1 ou plusieurs heures. Dans le cas où les étapes (a) et (c) sont décalées dans le temps, la composition contenant notamment un actif hydratant, apaisant ou anti-vieillissement est ensuite appliquée sur le visage et le cou et laissée en place pendant au moins 5 minutes, éventuellement pendant au moins une journée ou une nuit.

Le procédé peut également être à visée amincissante, et l'émulsion E/H selon l'invention est appliquée sur les zones du corps présentant où l'on souhaite réduire les amas graisseux, par exemple les cuisses, le ventre, les bras, le bas du visage, de façon simultanée, séparée ou séquencée dans le temps avec un actif cosmétique amincissant et/ou lipolytique.

Selon un autre aspect, le procédé est destiné à ralentir la chute des cheveux et/ou à augmenter le diamètre et/ou la densité de la tige pilaire, ou à diminuer la canitie: l'émulsion E/H selon l'invention est appliquée sous forme de masque, de crème ou de lotion capillaire de prétraitement du cuir chevelu; on applique ensuite la composition contenant l'actif, par exemple sous forme d'un shampooing qui est ensuite rincé, ou d'une lotion destinée à rester en contact avec le cuir chevelu pendant au moins une journée voire jusqu'au prochain shampooing.

Les actifs cosmétiques convenant à la mise en oeuvre de l'invention sont les actifs dont l'activité s'exerce dans des couches plus profondes que le stratum corneum, et qui ne doivent donc pas rester à la surface de la peau pour avoir une efficacité optimale.

On peut citer notamment les agents hydratants, les desquamants, anti-séborrhéiques, les agents anti-vieillissement, les dépigmentants ou anti-pigmentant, les pigmentant ou pro-pigmentant, les myorelaxants ou dermo-décontractants, les agents apaisants, les agents lipolytiques ou amincissant, les agents favorisant la microcirculation, les actifs diminuant chute et/ou favorisant la croissance des cheveux.

Les agents anti-vieillissement peuvent être choisis par exemple parmi les agents anti-radicaux libres, les agents anti-glycation, les inhibiteurs de NO synthase, les agents stimulant la synthèse de macromolécules dermiques ou épidermiques et/ou empêchant leur dégradation, les agents stimulant la prolifération des fibroblastes ou des kératinocytes et/ou empêchant la différenciation des kératinocytes, les agents kératolytiques, les vitamines, les agents anti-élastase et anti-collagénase, les dérivés d'acides gras, les oligoélements, les extraits d'algues et de planctons, les enzymes, coenzymes, les flavonoïdes, les agents myorelaxants.

Agents desquamants et hydratants

Par "agent desquamant", on entend tout composé capable d'agir :

- soit directement sur la desquamation en favorisant l'exfoliation, tel que les β-hydroxyacides, en particulier l'acide salicylique et ses dérivés (dont l'acide n-octanoyl 5-salicylique) ; les α-hydroxyacides, tels que les acides glycolique, citrique, lactique, tartrique, malique ou mandélique ; l'urée ; l'acide gentisique ; les oligofucoses ; l'acide cinnamique ; l'extrait de Saphora japonica ; le resvératrol et certains dérivés d'acide jasmonique ;

- soit sur les enzymes impliquées dans la desquamation ou la dégradation des cornéodesmosomes, les glycosidases, la stratum corneum chymotryptic enzym (SCCE) voire d'autres protéases (trypsine, chymotrypsine-like). On peut citer les agents chélatant des sels minéraux: l'EDTA ; l'acide N-acyl-N,N',N' éthylène diaminetriacétique ; les composés aminosulfoniques et en particulier l'acide (N-2 hydroxyéthylpiperazine-N-2-éthane) sulfonique (HEPES) ; les dérivés de l'acide 2-oxothiazolidine-4-carboxylique (procystéine) ; les dérivés d'acides alpha aminés de type glycine (tels que décrits dans EP-0 852 949, ainsi que le méthyl glycine diacétate de sodium commercialisé par BASF sous la dénomination commerciale TRILON M) ; le miel ; les dérivés de sucre tels que l'O-octanoyl-6-D-maltose et la N-acétyl glucosamine.

Par "agent hydratant", on entend notamment un composé agissant sur la fonction barrière, en vue de maintenir l'hydratation du stratum corneum. On peut citer les céramides, les composés à base sphingoïde, les lécithines, les glycosphingolipides, les phospholipides, le cholestérol et ses dérivés, les phytostérols (stigmastérol, β-sitostérol, campestérol), les acides gras essentiels, le 1-2 diacylglycérol, la 4-chromanone, les triterpènes pentacycliques tels que l'acide ursolique.

Ces composés peuvent représenter de 0,001% à 30%, et de préférence de 0,01 à 20%, du poids total de la composition selon l'invention.

La composition selon la présente invention comprenant les agents desquamants et hydratants cités ci-dessus est avantageusement destinée à la prévention ou au traitement du dessèchement de la peau et notamment des xéroses.

Agent dépigmentant, anti-pigmentant ou pro-pigmentant

- Comme agents blanchissants ou dépigmentants, on peut citer par exemple l'acide kojique et ses dérivés ; l'hydroquinone et ses dérivés tels que l'arbutine et ses esters ; la vitamine C et ses dérivés tels que l'ascorbyle phosphate de magnésium ; les sels tels que le calcium D pantéthéine sulfonate ; l'acide ellagique et ses dérivés ; le rucinol ; l'acide linoléique et ses dérivés ; les extraits de plantes, et notamment de réglisse, de mûrier ou de scutellaire et de Bacopa monnieri, sans que cette liste soit limitative; le glutathion et ses précurseurs ; la cystéine et ses précurseurs ; les composés dérivés d'aminophénol décrits dans le document WO-A-99/10318, comme notamment le N-éthyloxycarbonyl-4-amino-phénol, le N-éthyloxycarbonyl-O-éthyloxycarbonyl-4-amino-phénol, le N-cholestéryloxycarbonyl-4-aminophénol, le N-éthylamino-carbonyl-4-aminophénol ; et les mélanges de ces composés.

Comme agent pro-pigmentant, on peut citer l'extrait de pimprenelle (Sanguisorba officinalis) commercialisé par la société MARUZEN et les extraits de chrysanthème (Chrysanthemum morifolium).

La composition selon la présente invention comprenant les agents dépigmentants cités ci-dessus est avantageusement destinée à la prévention ou au traitement des hyperpigmentations, en particulier des taches pigmentaires liées au vieillissement de la peau.

De son côté, la composition renfermant les agents pro-pigmentants cités précédemment est de notamment destinée au traitement de la canitie, ou pour favoriser la pigmentation de la peau.

Agent anti-glycation

Par "agent anti-glycation", on entend un composé prévenant et/ou diminuant la glycation des protéines de la peau, en particulier des protéines du derme telles que le collagène.

Des exemples d'agents anti-glycation sont les extraits végétaux de la famille des Ericaceae, tels qu'un extrait de myrtille (*Vaccinium angusfifollium*) ; l'ergothionéine et ses dérivés ; et les hydroxystilbènes et leurs dérivés, tels que le resvératrol et le 3,3', 5,5'-tétrahydroxystilbène. Ces agents anti-glycation sont décrits dans les demandes FR 2 802 425, FR 2 810 548, FR 2 796 278 et FR 2 802 420, respectivement.

La composition selon l'invention comprenant un agent anti-glycation tel que défini ci-dessus peut avantageusement être utilisée pour prévenir ou traiter les signes du vieillissement cutané, en particulier pour prévenir ou traiter la perte de tonicité et/ou d'élasticité de la peau.

Inhibiteur de NO-synthase

Des exemples d'inhibiteurs de NO-synthase convenant à une utilisation dans la présente invention comprennent notamment un extrait de végétal de l'espèce *Vitis vinifera* qui est notamment commercialisé par la société Euromed sous la dénomination Leucocyanidines de raisins extra, ou encore par la société Indena sous la dénomination Leucoselect®, ou enfin par la société Hansen sous la dénomination Extrait de marc de raisin ; un extrait de végétal de l'espèce *Olea europaea* qui est de préférence obtenu à partir de feuilles d'olivier et est notamment commercialisé par la société VINYALS sous forme d'extrait sec, ou par la société Biologia & Technologia sous la dénomination commerciale Eurol BT ; et un extrait d'un végétal de l'espèce *Gingko biloba* qui est de préférence un extrait aqueux sec de ce végétal vendu par la société Beaufour sous le nom commercial Ginkgo biloba extrait standard.
La composition selon l'invention comprenant un inhibiteur de NO-synthase tel que défini ci-dessus peut avantageusement être utilisée pour prévenir ou traiter les signes du vieillissement cutané et/ou les peaux sensibles.

Agent anti-séborrhéique

Lorsque la composition selon l'invention comprend un agent anti-séborrhéique tel qu'un inhibiteur de 5α-réductase, celui-ci peut notamment être choisi parmi :

- les rétinoïdes, et en particulier le rétinol ;
- le soufre et les dérivés soufrés ;
- les sels de zinc tels que le lactate, le gluconate, le pidolate, le carboxylate, le salicylate et/ou le cystéate de zinc ;
- la vitamine B6 ou pyridoxine ;
- le mélange de capryloyl glycine, de sarcosine et d'extrait de cinnamomum zeylanicum commercialisé notamment par la société SEPPIC sous la dénomination commerciale Sepicontrol A5® ;
- un extrait de Laminaria saccharina commercialisé notamment par la société SECMA sous la dénomination commerciale Phlorogine® ;
- un extrait de Spiraea ulmaria commercialisé notamment par la société SILAB sous la dénomination commerciale Sebonormine® ;
- des extraits de végétaux des espèces Arnica montana, Cinchona succirubra, Eugenia caryophyllata, Humulus lupulus, Hypericum perforatum, Mentha piperita, Rosmarinus officinalis, Salvia oficinalis et Thymus vulgaris, tous commercialisés par exemple par la société MARUZEN ;
- un extrait de Serenoa repens commercialisé notamment par la société EUROMED ;
- des extraits de plantes du genre Silybum ;
- des extraits végétaux contenant des sapogénines et en particulier les extraits de Dioscorées riches en diosgénine ou hécogénine ; et
- des extraits d'Eugenia caryophyllata contenant de l'eugénol et du glucoside d'eugényle.

L'agent anti-séborrhéique représente par exemple de 0,001% à 10%, et de préférence de 0,01 à 5%, du poids total de la composition selon l'invention. Lorsque celle-ci renferme un tel composé, elle est particulièrement bien adaptée à prévenir ou traiter la séborrhée et/ou l'hirsutisme et/ou l'alopécie androgéno-dépendante.

Actif anti-chute inhibiteur de lysyl et/ou prolyl hydroxylase

Des exemples préférés d'inhibiteurs de lysyl et/ou propyl hydroxylase utilisables selon la présente invention sont le 2,4-diamino-pyrimidine 3-oxyde ou 2,4-DPO décrit dans la demande de brevet WO 96/09048 et le 2,4-diamino-6-pipéridino pyrimidine 3-oxyde ou "Minoxidil" décrit dans les brevets US-4,139,619 et US-4,596,812.
Ces composés sont par exemple présents dans la composition selon l'invention à hauteur de 0,001 à 5% en poids et, mieux, à hauteur de 0,01 à 5% en poids, par rapport au poids total de la composition.
La composition renfermant l'inhibiteur de lysyl et/ou prolyl hydroxylase est avantageusement utilisée pour la prévention ou le traitement de l'alopécie.

Agent stimulant la synthèse de macromolécules dermigues ou épidermiques et/ou empêchant leur dégradation

Parmi les actifs stimulant les macromolécules du derme ou empêchant leur dégradation, on peut citer ceux

qui agissent :

- soit sur la synthèse du collagène, tels que les extraits de Centella asiatica ; les asiaticosides et dérivés ; l'acide ascorbique ou vitamine C et ses dérivés ; les peptides de synthèse tels que la iamin, le biopeptide CL ou palmitoyloligopeptide commercialisé par la société SEDERMA ; les peptides extraits de végétaux, tels que l'hydrolysat de soja commercialisé par la société COLETICA sous la dénomination commerciale Phytokine® ; et les hormones végétales telles que les auxines et les lignanes.
- soit sur la synthèse d'élastine, tels que l'extrait de Saccharomyces Cerivisiae commercialisé par la société LSN sous la dénomination commerciale Cytovitin® ; et l'extrait d'algue Macrocystis pyrifera commercialisé par la société SECMA sous la dénomination commerciale Kelpadelie® ;
- soit sur la synthèse des glycosaminoglycanes, tels que le produit de fermentation du lait par lactobacillus vulgaris, commercialisé par la société BROOKS sous la dénomination commerciale Biomin yogourth® ; l'extrait d'algue brune Padina pavonica commercialisé par la société ALBAN MÜLLER sous la dénomination commerciale HSP3® ; et l'extrait de Saccharomyces cerevisiae disponible notamment auprès de la société SILAB sous la dénomination commerciale Firmalift® ou auprès de la société LSN sous la dénomination commerciale Cytovitin® ;
- soit sur la synthèse de la fibronectine, tels que l'extrait de zooplancton Salina commercialisé par la société SEPORGA sous la dénomination commerciale GP4G® ; l'extrait de levure disponible notamment auprès de la société ALBAN MÜLLER sous la dénomination commerciale Drieline® ; et le palmitoyl pentapeptide commercialisé par la société SEDERMA sous la dénomination commerciale Matrixil® ;
- soit sur l'inhibition des métalloprotéinases (MMP) telles que plus particulièrement les MMP 1, 2, 3, 9 . On peut citer : les rétinoïdes et dérivés, les oligopeptides et les lipopeptides, les lipoaminoacides, l'extrait de malt commercialisé par la société COLETICA sous la dénomination commerciale Collalift® ; les extraits de myrtille ou de romarin ; le lycopène ; les isoflavones, leurs dérivés ou les extraits végétaux en contenant, en particulier les extraits de soja (commercialisé par exemple par la société ICHIMARU PHARCOS sous la dénomination commerciale Flavostérone SB®), de trèfle rouge, de lin, de kakkon ou de sauge ;
- soit sur l'inhibition des sérine protéases telles que l'élastase leucocytaire ou la cathepsine G. On peut citer: l'extrait peptidique de graines de légumineuse (Pisum sativum) commercialisé par la société LSN sous la dénomination commerciale Parelastyl® ; les héparinoïdes ; et les pseudodipeptides tels que l'acide {2-[acétyl-(3-trifluorométhyl-phényl)-amino]-3-méthyl-butyrylamino} acétique.

Parmi les actifs stimulant les macromolécules épidermiques, telles que la fillagrine et les kératines, on peut citer notamment l'extrait de lupin commercialisé par la société SILAB sous la dénomination commerciale Structurine® ; l'extrait de bourgeons de hêtre Fagus sylvatica commercialisé par la société GATTEFOSSE sous la dénomination commerciale Gatuline® ; et l'extrait de zooplancton Salina commercialisé par la société SEPORGA sous la dénomination commerciale GP4G®.

La composition selon l'invention renfermant un ou plusieurs des composés ci-dessus convient particulièrement bien à une utilisation dans la prévention ou le traitement des signes cutanés du vieillissement, en particulier de la perte de fermeté et/ou d'élasticité de la peau.

Agent stimulant la prolifération des fibroblastes ou des kératinocytes et/ou la différenciation des kératinocytes

Les agents stimulant la prolifération des fibroblastes utilisables dans la composition selon l'invention peuvent par exemple être choisis parmi les protéines ou polypeptides végétaux, extraits notamment du soja (par exemple un extrait de soja commercialisé par la société LSN sous la dénomination Eleseryl SH-VEG 8® ou commercialisé par la société SILAB sous la dénomination commerciale Raffermine®) ; et les hormones végétales telles que les giberrellines et les cytokinines.

Les agents stimulant la prolifération des kératinocytes, utilisables dans la composition selon l'invention, comprennent notamment les rétinoïdes tels que le rétinol et ses esters, dont le palmitate de rétinyle ; l'adénosine ; le phloroglucinol ; les extraits de tourteaux de noix commercialisés par la société GATTEFOSSE ; et les extraits de Solanum tuberosum commercialisés par la société SEDERMA.

Les agents stimulant la différenciation des kératinocytes comprennent par exemple les minéraux tels que le calcium ; l'extrait de lupin commercialisé par la société SILAB sous la dénomination commerciale Photopréventine®; le beta-sitosteryl sulfate de sodium commercialisé par la société SEPORGA sous la dénomination commerciale Phytocohésine® ; et l'extrait de maïs commercialisé par la société SOLABIA sous la dénomination commerciale Phytovityl® ; et les lignanes tels que le sécoisolaricirésinol..

La composition selon l'invention comprenant ces composés est préférentiellement destinée à être utilisée pour prévenir ou traiter les signes cutanés du vieillissement.

Agent myorelaxant ou dermo-décontractant

Les agents myorelaxants ou dermo-décontractants selon l'invention comprennent l'alvérine et ses sels, le gluconate de manganèse, le Diazepam, l'hexapeptide Argireline R commercialisé par la société LIPOTEC, certaines amines secondaires et tertiaires carbonylées, l'adénosine, ainsi que les sapogénines et les extraits naturels, en particulier de Wild Yam, en contenant, ainsi que les extraits de Boswellia serrata.

Les agents anti-radicalaires utilisables dans la composition selon l'invention comprennent, la vitamine E et ses dérivés tels que l'acétate de tocophéryle; les bioflavonoïdes ; le co-enzyme Q10 ou ubiquinone ; certaines enzymes comme la catalase, le superoxyde dismutase et les extraits de germes de blé en contenant, la lactoperoxydase, le glutathion peroxydase et les quinones réductases ; le glutathion ; le benzylidène camphre ; les benzylcyclanones ; les naphtalénones substituées ; les pidolates ; le phytantriol ; le gamma-oryzanol ; la guanosine ; les lignanes ; et la mélatonine.

Agents apaisants

Comme agents apaisants utilisables dans la composition selon l'invention, on peut citer : les triterpènes pentacycliques et les extraits de plantes (ex : Glycyrrhiza glabra) en contenant comme l'acide β-glycyrrhétinique et ses sels et/ou ses dérivés (l'acide glycyrrhétinique monoglucuronide, le stearyl glycyrrhetinate, l'acide 3- stéaroyloxy glycyrrhetique), l'acide ursolique et ses sels, l'acide oléanolique et ses sels, l'acide bétulinique et ses sels, un extrait de Paeonia suffruticosa et / ou lactiflora, les sels de l'acide salicylique et en particulier le salicylate de zinc, les phycosaccharides de la société Codif, un extrait de Laminaria saccharina, l'huile de Canola, le bisabolol et les extraits de camomille, l'allantoïne, le Sépivital EPC (diesterphosphorique de vitamine E et C) de Seppic, les huiles insaturées en oméga 3 telles que les huiles de rosier muscat, de cassis, d'Echium, de poisson, des extraits de plancton, la capryloyl glycine, le Seppicalm VG (sodium palmitoylproline et nymphea alba) de Seppic, un extrait du Pygeum, un extrait de Boswellia serrata, un extrait de Centipeda cunnighami, un extrait d'Helianthus annuus, un extrait de Linum usitatissimum, les tocotrienols, les extraits de Cola nitida, le piperonal, un extrait de clou de girofle, un extrait d'Epilobium Angustifolium, l'aloe vera, un extrait de Bacopa monieri, les phytostérols, la cortisone, l'hydrocortisone, l'indométhacine et la beta méthasone.

les actifs lipolytiques ou ayant une activité favorable, directe ou indirecte, sur la diminution du tissu adipeux.

Parmi les dérivés susceptibles de favoriser la lipolyse on peut trouver :

1) les inhibiteurs de phosphodiestérase, tels que :

- les dérivés xanthiques comme la caféine et ses dérivés, notamment les 1-hydroxyalkylxanthines décrites dans le document FR-A-2,617,401, la caféine citrate, la théophylline et ses dérivés, la théobromine, l'acéfylline, l'aminophylline, le chloroéthylthéophylline, le diprofylline, le diniprophylline, l'étamiphylline et ses dérivés, l'étofylline, la proxyphylline ;
- les associations contenant des dérivés xanthiques, comme l'association de caféine et de silanol (dérivé méthylsilanetriol de caféine), et par exemple le produit commercialisé par la société Exsymol sous la dénomination caféisilane C ;
- les composés d'origine naturelle contenant des bases xanthiques et notamment de la caféine, tels que les extraits de thé, de café, de guarana, de maté, de cola (*Cola Nitida*) et notamment l'extrait sec de fruit de guarana (*Paulina sorbilis*) contenant 8 à 10 % de caféine ;
- l'éphédrine et ses dérivés qui peuvent notamment se retrouver à l'état naturel dans les plantes telles que le Ma Huang (Ephedra plant)

2) les extraits végétaux et les extraits d'origine marine, qui sont soit actifs sur les récepteurs à inhiber, tels que les β-2-bloqueurs, les NPY-bloqueurs (décrits dans le brevet EP 838217), soit inhibent la synthèse des récepteurs aux LDL ou VLDL, soit actifs pour stimuler les récepteurs β et les protéines G, conduisant à l'activation de l'adénylcyclase.

3) les peptides ou protéines

- les peptides dérivés de l'hormone parathyroidienne tels que décrits dans les brevets FR 2 788058 et FR 2781231 de Séderma ou les peptides décrits dans le document FR 2 786 693 voire tout autre peptide ayant des propriétés lipolytiques,
- les protamines et leurs dérivés tels que ceux décrits dans le document FR-A-2,758,724.

les agents agissant sur la microcirculation

Les actifs agissant sur la microcirculation (vasoprotecteur ou vasodilatateur) peuvent être choisis parmi les flavonoïdes, les ruscogénines, les esculosides, l'escine extraite du marron d'Inde, les nicotinates, l'héperidine méthyl chalcone, les huiles essentielles de lavande ou de romarin, les extraits de Ammi Visnaga.

La quantité de ces actifs peut varier dans une large mesure. De manière générale, ces actifs sont présents en une concentration allant de 0,01 à 15 % et de préférence de 0,05 à 10 % en poids par rapport au poids total de la composition.

Les agents agissant sur le métabolisme énergétique des cellules

Les actifs concernés sont ceux qui agissent sur le métabolisme énergétique cutané tel que, par exemple, et de façon non limitative, la synthèse d'ATP, ceux qui sont interviennent sur la chaine respiratoire de la cellule ou sur les réserves énergétiques. On peut citer le Coenzyme Q10 (ubiquinone), le cytochrome C, la créatine ou encore la phosphocréatine.

Des actifs particulièrement adaptés à la mise en oeuvre de l'invention sont ainsi les vitamines et en particulier le coenzyme Q10, la vitamine B3 et la vitamine C et ses dérivés, l'acide ellagique et de manière générale les dépigmentants, le rétinol et les rétinoïdes, les agents myorelaxants ou dermodécontractants, les actifs lipolytiques ou ayant une activité favorable sur la diminution du tissu adipeux.

Selon un des modes de réalisation avantageux de l'invention, le coefficient de partage octanol /eau du principe actif cosmétique exprimé en log P et calculé de manière connue par l'homme du métier, peut varier de -2 à +4.

L'invention a également pour objet une composition qui comprend (i) une émulsion eau dans huile comprenant une phase grasse de polarité δa supérieure ou égale à 0,1 et comportant un mélange d'au moins deux huiles telle que définie dans le présent texte et (ii) un actif cosmétique ou dermatologique, comme produit de combinaison pour une utilisation conjointe, simultanée, séparée ou étalée dans le temps en application topique. Ladite composition est notamment un produit de combinaison pouvant être sous forme de kit comportant les formulations (i) et (ii) correspondant aux compositions définies dans ce qui précède, et qui sont fournies à l'utilisateur qui applique les deux compositions de façon simultanée, séparée, ou étalée dans le temps. Le kit pourra se présenter sous forme de récipient comportant au moins un compartiment, et notamment au moins 2 compartiments contenant chacun respectivement au moins le composant (i) et au moins le composant (ii).

L'invention a également pour objet l'utilisation d'une composition sous forme d'une émulsion eau dans huile comprenant une phase grasse de polarité δa supérieure ou égale à 0,1 et comportant un mélange d'au moins deux huiles, telle que définie précédemment, pour favoriser la pénétration d'un actif cosmétique, et/ou pour la préparation d'une composition destinée à favoriser la pénétration d'un actif dermatologique.

Selon un mode de réalisation la composition sous forme d'émulsion E/H contiendra au moins un tensioactif choisi parmi les dérivés alkylés de polyglycérol, les polyéthylènes glycol alkylés, les dérivés alkylés de sorbitane, les sels métalliques d'acides gras, et les oligomères ou polymères dérivés de polyoléfines; de préférence elle contiendra au moins un tensioactif de type oligomère ou polymère dérivés de polyoléfines à terminaison succinique estérifiée.

Ladite composition sera utile pour favoriser la pénétration d'actifs définis dans ce qui précède, et notamment tels que des agents myorelaxant ou dermo-décontractants, d'agents dépigmentants et/ou d'agents stimulant la synthèse de macromolécules dermiques ou épidermiques. La dite composition et ledit actif cosmétique ou dermatologique pourront être appliqués conjointement, l'actif étant en mélange avec la composition E/H, ou de façon simultanée, décalée ou séquencée dans le temps.

En particulier, la composition E/H définie dans ce qui précède est une composition de prétraitement.

D'autres avantages de l'invention apparaîtront à la lecture des exemples qui suivent.

Exemple 1

Expérience d'absorption cutanée

*Formulations testées*

On prépare plusieurs compositions de type émulsion eau dans huile et comportant une phase grasse composée d'au moins deux huiles avec un δa particulier. Ces compositions sont exprimées en pourcentage massique.

| | A | B | C | D |
|---|---|---|---|---|
| A - AMMONIUM POLYACRYLOYLDIMETHYL TAURATE | 0,2 | 0,2 | 0,2 | 0,2 |
| sels | 2 | 2 | 2 | 2 |
| conservateurs | 0.2 | 0.2 | 0.2 | 0.2 |
| eau | qsp 100 | qsp 100 | qsp 100 | qsp 100 |
| B - POLYISOBUTYLENE A TERMINAISON SUCCINIQUE ESTERIFIE* | 1,92 | 1,92 | 1,92 | 1,92 |
| DIMETHICONE | 0 | 1,78 | 1,77 | 0,21 |
| SQUALANE | 0 | 2,66 | 2,65 | 0,32 |
| ISONONYL ISONONANOATE | 0 | 3,56 | 3,54 | 0,43 |
| HYDROGENATED POLYISOBUTENE | | | 0 | 7 |
| PRUNUS ARMENIACA | 15 | 7 | | |
| ZINC OXIDE | 0,5 | 0,5 | 0,5 | 0,5 |
| C- NYLON-12 | 3 | 3 | 3 | 3 |
| Polarité de la phase grasse($\delta a$ en $J^{1/2} cm^{-3/2}$) | 4.8 | 4.0 | 3.2 | 0.4 |

Mode opératoire :

Pour la phase A : homogénéiser l' AMMONIUM POLYACRYLOYLDIMETHYL TAURATE dans l'eau et ensuite mélanger avec tous les constituants de la phase A à température ambiante.

Mélanger et homogénéiser la phase B.

Ajouter lentement la phase A dans la phase B pour obtenir l'émulsion.

*référence commerciale utilisée: Lubrizol 5603 de la société Lubrizol

*Protocole d'évaluation*

L'étude est effectuée sur peau humaine entière. Les échantillons cutanés sont montés en cellules de diffusion en mode statique, de type « in-Line » de $0.5 cm^2$, pendant une durée totale de désorption de 16 heures.
Les formulations A, B, C et D sont appliquées à raison de 2 $mg/cm^2$ à la surface de la peau en tant que prétraitement. Une heure après cette application, une solution d'actif (caféine radiomarquée au $^{14}C$) est déposée. Une série d'échantillons cutanés de contrôle reçoit la solution de caféine directement sans prétraitement par la formulation.

Après l'application, la surface des échantillons est lavée. Seule l'analyse des excès de surface est réalisée en dosant la caféine $^{14}C$.
Les quantités de caféine [$^{14}C$] et/ou dérivés [$^{14}C$] récupérées dans les excès de surface sont exprimés en % de caféine / dose appliquée.

*Résultats*

| % excès de surface | A | B |
|---|---|---|
| avec prétraitement | 62,53 | 42,94 |
| sans prétraitement | 68,03 | 68,03 |
| % diminution de la caféine à la surface de la peau par rapport à l'échantillon sans prétraitement | -8,08 | -36,88 |

La comparaison des résultats obtenus pour les prétraitements avec les compositions cosmétiques A et B montrent que la composition B qui contient un mélange d'huile par rapport à la composition A qui ne contient qu'une seule huile permet une diminution plus importante de l'actif à la surface de la peau, soit une meilleur diffusion de la caféine au niveau de la peau.

| % excès de surface | C | D |
|---|---|---|
| avec prétraitement | 46,67 | 61,97 |
| sans prétraitement | 82,06 | 82,06 |
| % diminution de la caféine à la surface de la peau par rapport à l'échantillon sans prétraitement | -43,13 | -24,48 |

La comparaison des résultats obtenus pour les prétraitements avec les compositions cométiques C et D, montre que la composition C qui contient une phase huileuse avec un δa plus importante que la phase huileuse de la composition D permet un meilleur passage cutanée de l'actif.

Exemple 2:

Des formulations E et F, de type émulsion eau dans huile sont préparées selon les modalités décrites précédemment

| | E | F |
|---|---|---|
| AMMONIUM POLYACRYLOYLDIMETHYL TAURATE | 0,2 | 0,2 |
| sels | 2 | 2 |
| conservateurs | qs | qs |
| POLYISOBUTYLENE A TERMINAISON SUCCINIQUE ESTERIFIE | 1,92 | 1,92 |
| NYLON-12 | 3 | 3 |
| DIMETHICONE | 6,01 | |
| ZINC OXIDE | 0,5 | 0,5 |
| SQUALANE | 8,99 | |
| ISONONYL ISONONANOATE | | 15 |
| eau | qsp 100 | qsp 100 |

La pénétration de caféine après prétraitement est évaluée selon le protocole décrit à l'exemple 1

| % excès de surface | E | F |
|---|---|---|
| avec prétraitement | 60,87 | 83,85 |
| sans prétraitement | 85,88 | 85,88 |
| % diminution de la caféine à la surface de la peau par rapport à l'échantillon sans prétraitement | -29,12 | -2,36 |

La comparaison des résultats obtenus pour les prétraitements avec les compositions cosmétiques E et F montrent que la composition E qui contient un mélange d'huile par rapport à la composition F qui ne contient qu'une seule huile permet une diminution plus importante de l'actif à la surface de la peau, soit une meilleur diffusion de la caféine au niveau de la peau.

## Revendications

1. Procédé pour favoriser la pénétration d'un actif cosmétique **caractérisé en ce que** l'on applique topiquement une composition sous forme d'une émulsion eau dans huile, ladite émulsion comprenant une phase grasse de polarité δa supérieure ou égale à 0,1 et comportant un mélange d'au moins deux huiles, et **en ce que** l'application de ladite émulsion est réalisée de façon simultanée, décalée ou séquencée dans le temps avec l'application de l'actif cosmétique.

2. Procédé pour favoriser la pénétration d'un actif cosmétique selon la revendication 1, **caractérisé en ce que** la phase grasse de l'émulsion est un mélange d'au moins trois huiles, dont la polarité finale est supérieure à 0,1.

3. Procédé selon l'une au moins des revendications 1 ou 2, **caractérisé en ce que** la polarité de la phase grasse de l'émulsion est supérieure ou égale à 0,4.

4. Procédé selon l'une quelconque des revendications 1 à 3, **caractérisé en ce que** la phase grasse de l'émulsion comprend un mélange d'huiles apolaires et d'huiles polaires.

5. Procédé selon l'une quelconque des revendications précédentes, **caractérisé en ce que** la phase grasse de l'émulsion comprend de 20% à 80% p/p)) d'huiles polaires possédant des δa compris entre 2 et 11.

6. Procédé selon la revendication 5, **caractérisé en ce que** les polarités propres des huiles polaires présentes dans la phase grasse correspondent à des δa de 3 à 8.

7. Procédé selon l'une des revendications 5 ou 6, **caractérisé en ce que** la phase grasse comprend de 40% à 75% d'huiles polaires.

8. Procédé selon l'une quelconque des revendications précédentes, **caractérisé en ce que** la phase grasse de l'émulsion a une polarité δa inférieure ou égale à 8, en particulier inférieure ou égale à 4.

9. Procédé selon l'une quelconque des revendications précédentes, **caractérisé en ce que** l'émulsion comprend au moins une huile polaire choisie parmi les huiles de type silicone, les huiles végétales à forte teneur en triglycérides, les huiles de synthèse et/ou esters de synthèse.

10. Procédé selon la des revendication 9, **caractérisé en ce que** l'émulsion comprend au moins une huile choisie parmi les diméthicones, l'isononanoate d'isononyle, le lauroyl sarcosinate d'isopropyle et l'huile d'amandes d'abricot.

11. Procédé selon l'une quelconque des revendications précédentes, **caractérisé en ce que** la quantité de la phase grasse dans l'émulsion est de 0,01% à 50% en poids par rapport au poids total de la composition.

12. Procédé pour favoriser la pénétration d'un actif cosmétique selon l'une quelconque des revendications précédentes, **caractérisé en ce qu'**il comprend les étapes suivantes:

(a) on applique l'émulsion eau-dans-huile sur une zone de la peau et/ou du cuir chevelu,
(b) on laisse en contact l'émulsion avec la peau et/ou le cuir chevelu pendant un temps supérieur ou égal à 2 minutes,
(c) on applique l'actif cosmétique sur la même zone de la peau et/ou du cuir chevelu que celle de l'étape (a).

**13.** Procédé selon la revendication 12, **caractérisé en ce que** les étapes (a) et (c) sont effectuées simultanément, et **en ce que** l'actif cosmétique est présent dans la composition sous forme d'émulsion eau dans huile.

**14.** Procédé selon la revendication 12, **caractérisé en ce que** les étapes (a) et (c) sont effectuées de façon séquencée dans le temps, et **en ce que** l'actif cosmétique est présent dans une composition cosmétique comprenant un milieu physiologiquement acceptable.

**15.** Procédé selon l'une quelconque des revendications précédentes, **caractérisé en ce que** l'actif cosmétique est choisi parmi les agents hydratants, les desquamants, anti-séborrhéiques, les agents anti-vieillissement, les dépigmentants, les pro-pigmentant, les myorelaxants ou dermodécontractants, les apaisants, les agents lipolytiques ou amincissant, les agents favorisant la microcirculation, les actifs anti-chute des cheveux.

**16.** Procédé selon l'une quelconque des revendications précédentes, **caractérisé en ce que** l'émulsion eau dans huile comprend en outre au moins un tensioactif choisi parmi les dérivés alkylés de polyglycérol, les polyéthylènes glycol alkylés, les dérivés alkylés de sorbitane, les sels métalliques d'acides gras, les tensioactifs siliconés et les oligomères ou polymères dérivés de polyoléfines.

**17.** Procédé selon la revendication 16 **caractérisé en ce que** l'émulsion eau-dans-huile comprend au moins un tensioactif de type oligomère ou polymère dérivés de polyoléfines à terminaison succinique estérifiée.

**18.** Composition **caractérisée en ce qu'**elle comprend (i) une émulsion eau dans huile comprenant une phase grasse de polarité δa supérieure ou égale à 0,1 et comportant un mélange d'au moins deux huiles telle que définie dans l'une des revendications précédentes et (ii) un actif cosmétique ou dermatologique, comme produit de combinaison pour une utilisation simultanée, séparée ou étalée dans le temps en application topique.

**19.** Composition selon la revendication 18, **caractérisé en ce qu'**elle comprend au moins un tensioactif de type oligomère ou polymère dérivés de polyoléfines à terminaison succinique estérifiée.

**20.** Utilisation d'une composition sous forme d'une émulsion eau dans huile comprenant une phase grasse de polarité δa supérieure ou égale à 0,1 et comportant un mélange d'au moins deux huiles, pour favoriser la pénétration d'un actif cosmétique

Office européen des brevets

# RAPPORT DE RECHERCHE EUROPEENNE

Numéro de la demande
EP 05 29 1275

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X | US 2003/017176 A1 (BLECKMANN ANDREAS ET AL) 23 janvier 2003 (2003-01-23)<br>* page 2, alinéa 23 - alinéa 27 *<br>* page 2, alinéa 37 - page 3, alinéa 40 *<br>* page 5, alinéa 63 *<br>* exemples *<br>----- | 1-20 | A61K7/02<br>A61K7/40<br>A61K7/06 |
| X | EP 1 342 463 A (BEIERSDORF AG)<br>10 septembre 2003 (2003-09-10)<br>* page 2, alinéa 6 - page 3, alinéa 7 *<br>* page 3, alinéa 12 *<br>* page 6, alinéa 46 - page 7, alinéa 46 *<br>* exemples *<br>----- | 18 | |
| Y | US 2003/108579 A1 (YOUSFI NAIMA ET AL)<br>12 juin 2003 (2003-06-12)<br>* page 1, alinéa 7 *<br>* page 1, alinéa 14 - page 2, alinéa 15 *<br>* page 2, alinéa 23 - page 3, alinéa 43 *<br>* page 4, alinéa 51 *<br>* exemples *<br>----- | 1-20 | **DOMAINES TECHNIQUES RECHERCHES (Int.Cl.7)** |
| Y | US 5 489 429 A (GRIAT ET AL)<br>6 février 1996 (1996-02-06)<br>* colonne 1, ligne 4 - ligne 10 *<br>* colonne 2, ligne 64 - colonne 3, ligne 19 *<br>* exemples *<br>----- | 1-20 | A61K |

Le présent rapport a été établi pour toutes les revendications

2

| Lieu de la recherche | Date d'achèvement de la recherche | Examinateur |
|---|---|---|
| Munich | 29 septembre 2005 | Ruckebusch, V |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
..........................................................................................
& : membre de la même famille, document correspondant

EPO FORM 1503 03.82 (P04C02)

**ANNEXE AU RAPPORT DE RECHERCHE EUROPEENNE RELATIF A LA DEMANDE DE BREVET EUROPEEN NO.** EP 05 29 1275

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

29-09-2005

| Document brevet cité au rapport de recherche | Date de publication | Membre(s) de la famille de brevet(s) | Date de publication |
|---|---|---|---|
| US 2003017176 A1 | 23-01-2003 | DE 19924277 A1 | 30-11-2000 |
| | | EP 1055424 A2 | 29-11-2000 |
| | | JP 2001002525 A | 09-01-2001 |
| EP 1342463 A | 10-09-2003 | DE 10210337 A1 | 25-09-2003 |
| US 2003108579 A1 | 12-06-2003 | CA 2406365 A1 | 25-04-2003 |
| | | CN 1413568 A | 30-04-2003 |
| | | EP 1306074 A1 | 02-05-2003 |
| | | FR 2831433 A1 | 02-05-2003 |
| | | JP 2003137724 A | 14-05-2003 |
| US 5489429 A | 06-02-1996 | CA 2134623 A1 | 30-04-1995 |
| | | EP 0650717 A1 | 03-05-1995 |
| | | FR 2711530 A1 | 05-05-1995 |
| | | JP 7256085 A | 09-10-1995 |

EPO FORM P0460

Pour tout renseignement concernant cette annexe : voir Journal Officiel de l'Office européen des brevets, No.12/82